# EUROPEAN PATENT APPLICATION

(11) **EP 3 252 632 A1**
(43) Date of publication of application: **06.12.2017**
(21) Application number: 17171247.4
(22) Date of filing: 16.05.2017
(51) Int. Cl.: G06F 19/00

(54) **TREATMENT SYSTEM INCLUDING MASSAGE MACHINE**

(30) Priority: 03.06.2016 JP 2016111868
(71) Applicant: Family Inada Co., Ltd., Osaka, Osaka 532-0004 (JP)
(72) Inventor: INADA, Nichimu, Osaka, Osaka 532-0004 (JP); ISHIDOU, Yuta, Saihaku-gun, Tottori 689-3224 (JP); NOTSU, Yuji, Saihaku-gun, Tottori 689-3224 (JP); SASAKI, Izumi, Saihaku-gun, Tottori 689-3224 (JP)
(74) Representative: Horn Kleimann Waitzhofer Patentanwälte PartG mbB

(57) **Abstract**

Provided is a treatment system including a massage machine which recommends a suitable treatment course to a treatment subject based on body information of the treatment subject measured by a measurement portion before treatment. There are provided a measurement portion which measures body information of a treatment subject before treatment; a massage machine which has a treatment portion treating the treatment subject, a control unit controlling the treatment portion, and a storage portion storing a plurality of treatment courses having treatment contents for the treatment portion different from each other; and a notification portion which recommends the treatment courses to the treatment subject. The measurement portion and the control unit are connected to each other through a cable or by radio. The control unit is configured to receive the body information of the treatment subject from the measurement portion through a cable or by radio, and to select a suitable treatment course from the plurality of treatment courses stored in the storage portion, based on the body information such that the notification portion recommends the selected treatment course to the treatment subject.

## Description

### TECHNICAL FIELD

The present invention relates to a treatment system including a massage machine.

### BACKGROUND ART

In the related art, a treatment subject treats each site of the body by using a massage machine. In addition, for his/her own health management, the treatment subject conducts health management by using various types of measurement instruments, such as blood pressure management using a sphygmomanometer, and weight management using a weight scale. In this manner, generally, treatment of the body using a massage machine and health management using a measurement instrument have been separately conducted.

Meanwhile, there is a health improvement system in which a function of the massage machine is restricted based on information from the measurement instrument (for example, PTL 1). In the health improvement system, based on the information from the measurement instrument, when the health condition of the treatment subject is determined to be more favorable than a standard, the operation is not restricted, and when the health condition thereof is determined to be less favorable than the standard, the operation is restricted.

In addition, as another technology in the related art, there is a massage apparatus in which when a treatment subject inputs an answer to a questionnaire from stimulus inquiry means of the massage apparatus, a stronger stimulus or a weaker stimulus is applied to an acupuncture point in accordance with the answer (for example, refer to PTL 2).

Moreover, there is further another technology in the related art in which information recommended for a next operation of a massage machine is output to a notification portion based on biophysiological information of a treatment subject changing in response to a massage (for example, refer to PTL 3).

### RELATED ART DOCUMENT

### PATENT DOCUMENT

- Patent Document 1:: JP-A-2009-112338
- Patent Document 2:: JP-A-2002-238965
- Patent Document 3:: JP-A-2016-36648

### SUMMARY OF THE INVENTION

### PROBLEM THAT THE INVENTION IS TO SOLVE

However, in PTL 1, the function of the massage machine is restricted by a control unit in accordance with the health condition of the treatment subject, and the treatment subject cannot select treatment contents. Therefore, in this technology in the related art, the treatment subject cannot select treatment suitable for himself/herself.

In addition, in PTL 2, the massage apparatus adjusts stimulus intensity based on the answer of the inquiry result with respect to a user. Therefore, in this technology in the related art as well, the treatment subject cannot select treatment suitable for himself/herself.

Moreover, in PTL 3, a next operation is recommended based on the biophysiological information changing in response to a massage. However, in this technology in the related art, it is not possible to know a treatment course suitable for body information of the treatment subject changing day by day, before using the massage machine.

An object of the present invention is to provide a treatment system including a massage machine which recommends a suitable treatment course to a treatment subject based on body information of the treatment subject measured by a measurement portion before treatment.

### MEANS FOR SOLVING THE PROBLEM

In order to achieve the object, according to the present invention, there is provided a treatment system including a measurement portion which measures body information of a treatment subject before treatment; a massage machine which has a treatment portion treating the treatment subject, a control unit controlling the treatment portion, and a storage portion storing a plurality of treatment courses having treatment contents for the treatment portion different from each other; and a treatment course notification portion which recommends the treatment courses to the treatment subject. The measurement portion and the control unit are connected to each other through a cable or by radio. The control unit is configured to receive the body information of the treatment subject from the measurement portion through a cable or by radio, and to select a suitable treatment course from the plurality of treatment courses stored in the storage portion, based on the body information such that the treatment course notification portion recommends the selected treatment course to the treatment subject.

According to the configuration, when the treatment subject measures body information by using the measurement portion before treatment, the control unit of the massage machine causes the treatment course notification portion to recommend a treatment course suitable for the body information of the treatment subject based on the body information. Thus, the treatment subject can easily recognize the treatment course suitable for his/her own body information before treatment and can execute the treatment course.

In addition, the control unit may be configured to be able to change at least any one of a massaging force and a part of a treatment operation in the treatment course based on the body information of the treatment subject.

According to such a configuration, the massaging force and the treatment operation can be changed based on the body information of the treatment subject. For example, in a case where the physical condition of the treatment subject is found to be not favorable based on the body information, treatment can start from a state where the massage force is weakened or can start from a state where a part of the treatment operation is turned off.

In addition, the measurement portion may have at least a sphygmomanometer. The plurality of treatment courses may include at least a blood circulation improvement treatment course for improving blood circulation. The control unit may be configured to determine whether or not to select the blood circulation improvement treatment course as the suitable treatment course, based on the body information of the treatment subject from the sphygmomanometer. The "blood circulation improvement treatment course" is acceptable as long as the treatment course includes treatment related to blood circulation. For example, it is possible to include treatment and the like stimulating an acupuncture point of enhancing elasticity of a blood vessel. The "acupuncture point" in the documents of this specification and Claims is a so-called "pressure point".

According to such a configuration, the blood circulation improvement treatment course can be recommended as a treatment course suitable for the treatment subject based on a blood pressure value of the treatment subject measured by the sphygmomanometer. The blood circulation improvement treatment course can include a treatment course for a case where the blood pressure value is high and a treatment course for a case where the blood pressure value is low.

In addition, a blood pressure information notification portion may be further included. The control unit may be configured to cause the body information of the treatment subject from the sphygmomanometer to be chronologically recorded in the storage portion and to cause the blood pressure information notification portion to issue a notification of the chronological body information recorded in the storage portion.

According to such a configuration, since the blood pressure information notification portion issues a notification of a change in the body information measured by the treatment subject using the sphygmomanometer, the treatment subject can chronologically know the change of his/her own body information. Thus, the treatment subject can select the treatment course while grasping the change in his/her own body information.

In addition, the measurement portion may have at least a weight scale. The plurality of treatment courses may include at least a weight management treatment course for managing a weight. The control unit may be configured to determine whether or not to select the weight management treatment course as the suitable treatment course, based on the body information of the treatment subject from the weight scale. The "weight management treatment course" is acceptable as long as the treatment course includes treatment suitable for managing the weight. For example, it is possible to include treatment and the like stimulating an acupuncture point for promoting appetite or an acupuncture point for suppressing appetite.

According to such a configuration, the weight management treatment course can be recommended as a treatment course suitable for the treatment subject based on the weight of the treatment subject measured by using the weight scale. The weight management treatment course may include a treatment course for a case where the weight is gained and a treatment course for a case where the weight is lost.

In addition, a weight information notification portion may be further included. The control unit may be configured to cause the body information of the treatment subject from the weight scale to be chronologically recorded in the storage portion and to cause the weight information notification portion to issue a notification of the chronological body information recorded in the storage portion.

According to such a configuration, since the weight information notification portion issues a notification of a change in the body information measured by the treatment subject using the weight scale, the treatment subject can chronologically know the change of his/her own body information. Thus, the treatment subject can select the treatment course while grasping the change in his/her own body information.

In addition, an information terminal which is provided between the measurement portion and the control unit and in which the body information of the treatment subject is recorded may be further included. The information terminal may be configured to be able to communicate with the control unit through a cable or by radio. The "information terminal" in the documents of this specification and Claims includes a tablet terminal, a portable telephone, a wearable terminal, and the like.

According to such a configuration, the treatment subject can check his/her own body information through the information terminal even at a place separated from the massage machine. The control unit can receive the body information of the treatment subject through the information terminal.

In addition, the massage machine may have at least a seat portion in which the treatment subject takes a seat, and a backrest portion which supports the upper half of the body of the treatment subject who takes a seat in the seat portion, from the back. The control unit may be configured to set an erect/tilt angle of the backrest portion in accordance with the treatment course.

According to such a configuration, the erect/tilt angle of the backrest portion can be adjusted in accordance with the body information of the treatment subject. For example, in a case where the blood pressure value of the treatment subject is found to be high based on the body information, treatment can be weakened by narrowing the angle of the backrest portion such that the backrest portion is erected. In a case where the blood pressure value of the treatment subject is low, treatment can be strengthened by widening the angle of the backrest portion such that the backrest portion is tilted.

In addition, the massage machine may have a timepiece portion. The control unit may be configured to cause an alarm notification portion to issue a notification of an alarm at a predetermined time based on time information from the timepiece portion.

According to such a configuration, for example, in a case of using a treatment course related to blood pressure, it is possible to know a using time through the alarm notification portion issuing a notification of an alarm twice a day (morning and evening) at regular times. In addition, for example, in a case of using a treatment course related to weight, it is possible to know the using time through the alarm notification portion issuing a notification of an alarm once a day at a regular time.

In addition, the alarm notification portion may be configured to issue a notification of an alarm through at least one of light, a sound, vibration, and a screen display.

According to such a configuration, the treatment subject can easily know the using time of the massage machine through any alarm of light, a sound, vibration, and a screen display. Moreover, in a case where a plurality of treatment subjects use the massage machine, each of the plurality of treatment subjects can easily know his/her own alarm by causing the color of the light, the sound, and the screen display to be different depending on the treatment subject.

In addition, the control unit may be configured to recommend that the massage machine is not to be in use in a case where the body information of the treatment subject exceeds a recommended usage range.

According to such a configuration, the treatment subject can know that his/her own body information is within a range which is not suitable for using the massage machine. For example, in a case where the blood pressure value exceeds the recommended usage range, it is recommended that the massage machine is not to be in use. For example, the recommended usage range can be a range of a pulse value, in addition to the blood pressure value.

In addition, the control unit may be configured to cause a health information notification portion to issue a notification of advice related to health information prepared based on the body information of the treatment subject.

According to such a configuration, the treatment subject can check the health information prepared based on his/her own body information, through the advice given by the health information notification portion. For example, the advice on health management suitable for the treatment subject can be checked based on a change in the blood pressure value or a change in a weight value. For example, the advice can include a diagnosis result from a physician. The treatment subject can know information regarding his/her own health management when using the massage machine.

In addition, the massage machine may be connected to a hospital via a communication network. The control unit may ask the treatment subject a question related to the health information and transmit a result of the question to the communication network. The hospital may receive the result of the question from the communication network and transmit evaluation and advice with respect to the result of the question to the communication network. The control unit may be configured to receive the evaluation and advice from the communication network and to cause the health information notification portion to issue a notification.

According to such a configuration, it is possible to receive the evaluation and advice from the hospital with respect to the result of the question related to the health information conducted in the massage machine. The evaluation and advice is notified to the treatment subject by the health information notification portion and can be checked by the treatment subject.

In addition, the massage machine may have a camera imaging the treatment subject, and a microphone converting a voice of the treatment subject into an electric signal. The health information notification portion may also serve as a display portion imaging a physician of the hospital via the communication network. The health information notification portion may be configured to allow the treatment subject and the physician to have an interview.

According to such a configuration, the treatment subject can have an interview with a physician at a separated place via the health information notification portion of the massage machine.

In addition, the massage machine may be connected to a registered transmission destination via the communication network. The control unit may be configured to transmit the body information of the treatment subject to the registered transmission destination via the communication network. The "registered transmission destination" includes a service provider managing the massage machine, a communication server, a registerer terminal, and the like.

According to such a configuration, when the treatment subject measures the body information by using the measurement portion and the control unit receives the body information, the body information of the treatment subject can be transmitted to the registered transmission destination via the communication network.

### ADVANTAGE OF THE INVENTION

According to the present invention, the massage machine recommends a treatment course suitable for body information of a treatment subject based on body information of the treatment subject measured by the measurement portion before treatment. The treatment subject can select a treatment course suitable for himself/herself based on the recommendation.

### BRIEF DESCRIPTION OF THE DRAWINGS

- Fig. 1: is the overall configuration diagram illustrating a treatment system according to an embodiment of the present invention.
- Fig. 2: is a perspective view of a massage machine illustrated in Fig. 1.
- Fig. 3: is a functional block diagram of the massage machine illustrated in Fig. 2.
- Fig. 4: is a front view illustrating an example of a screen of an operation unit illustrated in Fig. 2.
- Fig. 5: is a front view illustrating an example of body information displayed in a blood pressure information notification portion in the screen of the operation unit illustrated in Fig. 2.
- Fig. 6: is a front view illustrating an example of body information displayed in a weight information notification portion in the screen of the operation unit illustrated in Fig. 2.
- Fig. 7: is a front view illustrating an example of the screen in which an alarm is set by using the operation unit illustrated in Fig. 2.
- Fig. 8: is a front view illustrating an example of a question conducted by using the operation unit illustrated in Fig. 2.

### BEST MODE FOR CARRYING OUT THE INVENTION

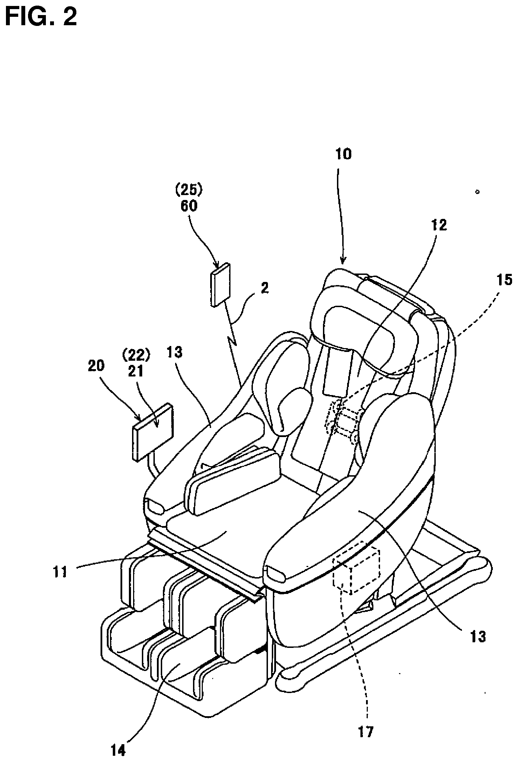

Hereinafter, an embodiment of the present invention will be described based on the drawings. In the embodiment below, description will be given regarding an example in which a massage machine 10, a sphygmomanometer 31 which is a measurement portion 30, and a weight scale 32 can communicate with each other through radio communication 2 (Bluetooth (registered trademark) or the like). In addition, in the embodiment, description will be given regarding an example in which a service provider 40 has a cloud-type communication server 41. The communication server 41 may be included in the service provider 40. The concept of forward, rearward, leftward, and rightward directions in the documents of this specification and Claims coincides with the concept of directions viewed from a treatment subject when the treatment subject takes a seat in a seat portion 11 of the massage machine 10 illustrated in Fig. 2.

### Configuration of Treatment System

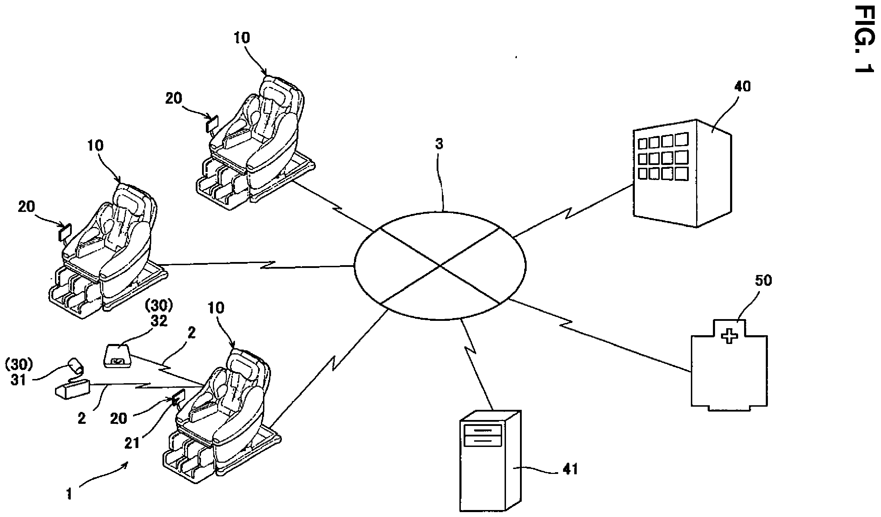

As illustrated in Fig. 1, a treatment system 1 including the massage machine 10 of the embodiment includes the massage machine 10 treating the treatment subject, and the measurement portion 30 measuring body information of the treatment subject. The measurement portion 30 of the embodiment includes the sphygmomanometer 31 and the weight scale 32 which are configured to be separate from the massage machine 10. The sphygmomanometer 31 and the weight scale 32 can communicate with the massage machine 10 through the radio communication 2. The measurement portion 30 may be connected to the massage machine 10 by radio or through a cable of different type. In addition, the measurement portion 30 may be built in the massage machine 10.

The treatment system 1 of the embodiment is connected to a communication network 3. The communication server 41 can communicate with the massage machine 10 via the communication network 3. For example, examples of the communication network 3 include the internet network, a communication network in a building, and a local network.

In addition, the communication server 41, the service provider 40, and a hospital (medical facility) 50 are connected to the communication network 3. In this example, an example of a cloud-type communication server 41 is illustrated. The communication server 41 may be a communication server 41 managed by the service provider 40 or may be a communication server 41 managed by a different service provider 40. For example, examples of the service provider 40 include a service provider 40 providing rental service of the massage machine 10, and a service provider 40 managing the massage machine 10 which has been sold.

For example, examples of the hospital 50 include a hospital 50 that is affiliated with the service provider 40 and provides advice related to health based on the body information of the treatment subject. The hospital 50 and the service provider 40 may be one body. The service provider 40 and the hospital 50 are not necessarily connected to each other via the communication network 3. According to the configuration including the hospital 50, as described below, the service provider 40 can provide the hospital 50 with the body information of the treatment subject. The hospital 50 can provide the treatment subject with advice based on the body information of the treatment subject. The advice can be transmitted from the service provider 40 to the massage machine 10 via the communication network 3 and can be provided for the treatment subject.

In the embodiment, the measurement portion 30 (sphygmomanometer 31 and weight scale 32) and the massage machine 10 (control unit 17 to be described below) can communicate with each other. An information terminal for recording the body information of the treatment subject may be additionally provided therebetween. As the information terminal, a tablet terminal, a portable telephone, a wearable terminal, or the like can be used. The information terminal is configured to be able to communicate with the control unit 17 through a cable or by radio.

According to such a configuration, the treatment subject can check his/her own body information through the information terminal even at a place separated from the massage machine 10. The control unit 17 receives the body information of the treatment subject through the information terminal. As described below, the control unit 17 can recommend a treatment course based on the body information to the treatment subject.

### Configuration of Massage Machine

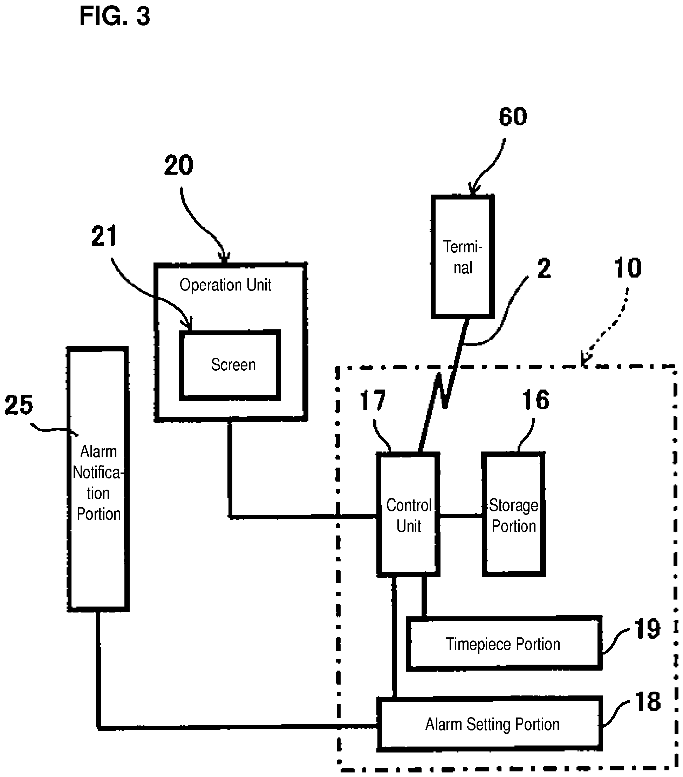

Fig. 2 is a perspective view of the massage machine 10. Fig. 3 is a functional block diagram of the massage machine 10. As illustrated, the massage machine 10 has the seat portion 11 in which the treatment subject takes a seat, and a backrest portion 12 which supports the upper half of the body of the treatment subject who takes a seat in the seat portion 11, from the back. The seat portion 11 has armrests 13 at the right and left positions. The seat portion 11 has a footrest 14 in the front of the seat portion 11. In this example, a treatment portion (roller unit) 15 of the backrest portion 12 is illustrated. Other portions are also provided with a treatment portion such as an air bag. The treatment portion 15 is controlled by the control unit 17. A plurality of treatment courses (will be described later) are set from combinations of the treatment portion 15 and are stored in a storage portion 16. In addition, in the storage portion 16, the body information of the treatment subject from the sphygmomanometer 31, and the body information of the treatment subject from the weight scale 32 (will be described later) are chronologically recorded by the control unit 17.

The massage machine 10 is provided with the control unit 17 which controls an operation of the treatment portion 15, an erect/tilt angle of the backrest portion 12 according to the treatment course (will be described later), and the like; and the storage portion 16 which stores the plurality of treatment courses having treatment contents for the treatment portion 15 different from each other. For example, the control unit 17 has a CPU, a ROM, a RAM, and the like. For example, as the storage portion 16, a flash memory, a hard disk drive, or the like can be used.

In addition, the massage machine 10 of the embodiment includes an alarm setting portion 18. The massage machine 10 has a timepiece portion 19. As described below, for example, the time for an alarm can be set or an alarm can be set to be emitted at a set time by the alarm setting portion 18. The alarm setting portion 18 can be included in the control unit 17. The control unit 17 emits an alarm at a predetermined time set through the alarm setting portion 18, based on time information of the timepiece portion 19.

In a portion of the armrest 13, there is provided an operation unit 20 for operating the massage machine 10. The operation unit 20 has a screen 21 which can be visually checked by the treatment subject. A treatment operation of the treatment portion 15 or the like is operated by using the operation unit 20. The operation unit 20 is provided so as to be able to be operated in a state where the treatment subject takes a seat. The operation unit 20 can be an operation unit 20 such as a tablet terminal. The control unit 17 is connected to the operation unit 20. In the embodiment, as described below, the screen 21 of the operation unit 20 has a function of a treatment course notification portion 22 which recommends a treatment course selected by the control unit 17 of the massage machine 10 to the treatment subject.

In addition, the screen 21 of the operation unit 20 has a function of a blood pressure information notification portion 23 and a function of a weight information notification portion 24 (will be described later). Moreover, the screen 21 of the operation unit 20 also has a function of an alarm notification portion 25 and a function of a health information notification portion 26.

The treatment course notification portion 22, the blood pressure information notification portion 23, the weight information notification portion 24, the alarm notification portion 25, and the health information notification portion 26 may be provided through a device other than the screen 21 of the operation unit 20, for example, a terminal 60 and a portable telephone independently allowing the radio communication 2 separately from the massage machine 10. When the terminal 60 has the alarm notification portion 25, the treatment subject can know an alarm even if the treatment subject is separated from the massage machine 10.

In addition, the treatment course notification portion 22, the blood pressure information notification portion 23, the weight information notification portion 24, and the health information notification portion 26 can issue a notification through a voice in addition to a screen display.

Moreover, the notification from the alarm notification portion 25 may be at least one of notifications of light, a sound, vibration, and a screen display. It is possible to more clearly notify the treatment subject of an alarm by combining two or more notification methods.

### Action of Treatment System

According to the massage machine 10, the body information of the treatment subject can be received by radio (through a cable) from at least any one of the sphygmomanometer 31 and the weight scale 32 which serve as the measurement portion 30. The control unit 17 of the massage machine 10 causes the treatment course notification portion 22 to recommend a suitable treatment course to the treatment subject from the plurality of treatment courses stored in the storage portion 16, based on the received body information. In the embodiment, recommendation is given through the treatment course notification portion 22 set in the screen 21 of the operation unit 20.

As the treatment course to be recommended, for example, a blood circulation improvement treatment course can be recommended to the treatment subject who has measured the body information by using the sphygmomanometer 31, based on the measured body information. A weight management treatment course can be recommended to the treatment subject who has measured the body information by using the weight scale 32, based on the measured body information. Moreover, for example, a suitable treatment course can be recommended in accordance with the body information such as a heart rate.

### Arrangement of Treatment Course

In addition, in each treatment course, the treatment contents can be arranged. The treatment course can be arranged by causing at least any one of a massaging force and a part of the treatment operation in each treatment course to be changeable. The arrangement is performed by the control unit 17 of the massage machine 10 based on the body information of the treatment subject. The fact that the treatment contents is arranged based on a measurement result of the body information is caused to be notified through the screen 21 of the operation unit 20 by the control unit 17.

Accordingly, the massaging force and the treatment operation can be changed based on the body information of the treatment subject. For example, in a case where the physical condition of the treatment subject is found to be not favorable base on the body information, treatment can start from a state where the massage force is weakened or can start from a state where a part of the treatment operation is turned off.

In addition, the control unit 17 can adjust the erect/tilt angle of the backrest portion 12 in accordance with the body information of the treatment subject. For example, in a case where the blood pressure of the treatment subject is found to be high based on the body information, treatment can be weakened by narrowing the angle of the backrest portion 12 such that the backrest portion 12 is erected. In addition, in a case where the blood pressure of the treatment subject is found to be low, treatment can be strengthened by widening the angle of the backrest portion 12 such that the backrest portion 12 is tilted.

### Treatment Course

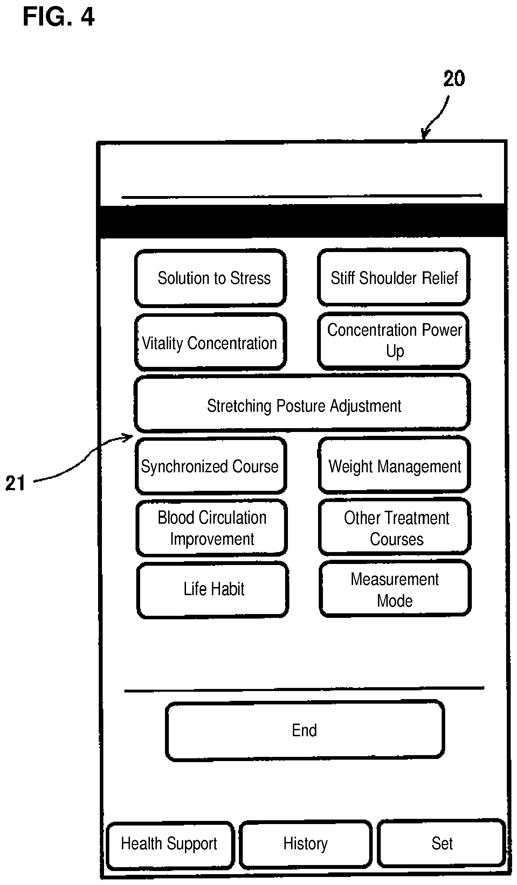

Fig. 4 is a front view illustrating an example of the screen 21 of the operation unit 20. As illustrated, the screen 21 of the operation unit 20 displays the treatment course stored in the storage portion 16. This example includes the plurality of treatment courses as follows.

Solution to stress: For example, this course can include treatment of lowering the pulse rate.

Stiff shoulder relief: This course provides treatment for shoulders and can be mainly kneading and patting treatment for shoulders.

Vitality concentration: This course can include treatment of simultaneously stimulating sites of the body with a plurality of treatment portions such that the body can be recovered from fatigue within a short period of time.

Concentration power up: This course can include treatment with a weak stimulus such that concentration power is enhanced after the treatment.

Stretching posture adjustment: For example, this course can include treatment of stretching muscles of the body.

Synchronized course: This course can include treatment associated with music so as to realize a stress reducing effect of music and a blood circulation promoting effect.

Weight management: This course includes treatment suitable for managing the weight. For example, this course can include treatment of stimulating an acupuncture point for promoting appetite or an acupuncture point for suppressing appetite and can include treatment of stretching each site.

Blood circulation improvement: This course can include treatment mainly for an acupuncture point for stabilizing blood circulation. For example, this course can include treatment of stimulating an acupuncture point for enhancing elasticity of blood vessels.

Life habit: This course mainly provides treatment in accordance with the activity time zone of the treatment subject. For example, in a case where the treatment subject acts in the morning, it is possible to perform treatment of stimulating an acupuncture point suitable for treatment in the morning. In a case where the treatment subject acts during the daytime, it is possible to mainly perform treatment of stretching, and in a case where the treatment subject acts at night, it is possible to perform treatment of stimulating an acupuncture point suitable for treatment at night.

In addition to the treatment courses, other treatment courses may be included. The plurality of treatment courses are not limited to the treatment courses. The plurality of treatment courses are stored in the storage portion 16 provided in the massage machine 10 and each treatment course is executed by the control unit 17. Hereinafter, the blood circulation improvement treatment course and the weight management treatment course will be described in detail.

### Blood Circulation Improvement Treatment Course

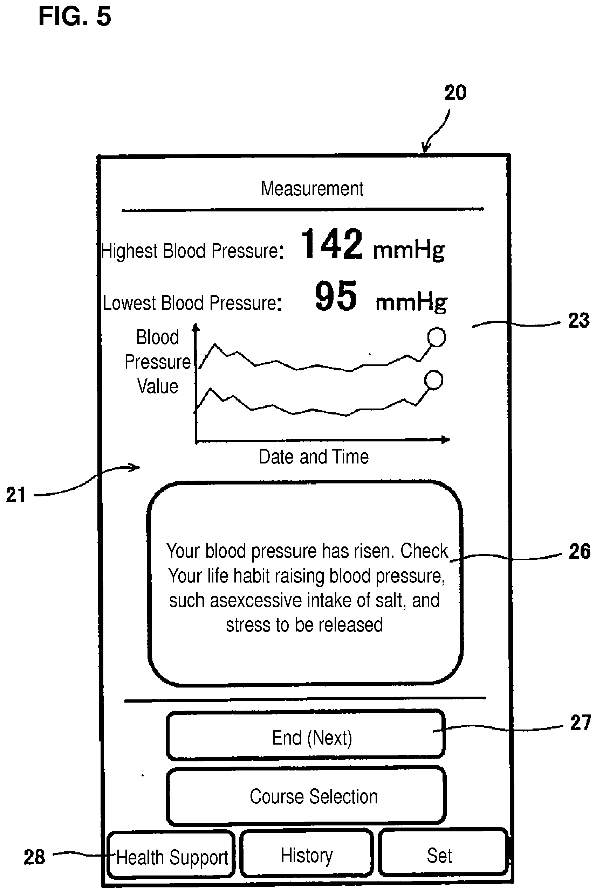

Fig. 5 is a front view illustrating an example of a screen for the measurement result displayed in the screen 21 of the operation unit 20. In this example, a result of a measurement performed by the treatment subject using the sphygmomanometer 31 before treatment is received by the control unit 17 of the massage machine 10, and the result is displayed in an upper portion of the screen 21 of the operation unit 20. Below thereof, chronological information of a blood pressure value (body information) of the treatment subject recorded in the storage portion 16 is displayed. In this example, a change in one week is graphically displayed.

In this manner, in a case where the treatment subject uses the sphygmomanometer 31 as the measurement portion 30 and the body information of the treatment subject is the blood pressure value, the lowest blood pressure and the highest blood pressure are shown. Accordingly, the treatment subject can grasp the change in his/her own body information through the operation unit 20. The portion of displaying information related to blood pressure is the blood pressure information notification portion 23. In this example, a part of the screen 21 serves as the blood pressure information notification portion 23. This example illustrates a case where the blood pressure value measured by the sphygmomanometer 31 is a high blood pressure value.

In addition, below the blood pressure information notification portion 23, advice given based on a chronological change in the blood pressure value is displayed. For example, the advice can be notified while including a diagnosis result from a physician (will be described later). In this manner, the portion of notifying the treatment subject of advice and the like given based on the body information of the treatment subject is the health information notification portion 26. In this example, a part of the screen 21 serves as the health information notification portion 26. Accordingly, the treatment subject can check suitable advice on the health management based on the change in the blood pressure value. When using the massage machine 10, the treatment subject can grasp activity to be performed for his/her own health management from the information of the health information notification portion 26.

Moreover, in a case of high blood pressure as in the example, it is possible to recommend that the massage machine 10 is not to be in use. As a reference value of high blood pressure, for example, the reference value of WHO (140 mmHg or higher) can be set as a recommended usage range. In a case of this example, since the highest blood pressure exceeds the recommended usage range, using the massage machine 10 can be ended by causing an [End] button 27 in the screen 21 to be flickered or the like.

In a case where the blood pressure value obtained from the sphygmomanometer 31 does not exceed a predetermined value, the [End] button 27 displays [Next], and when the button 27 is pressed, the blood circulation improvement treatment course is recommended as a suitable treatment course. The blood circulation improvement treatment course can include treatment mainly for an acupuncture point for enhancing elasticity of blood vessels, and treatment for relaxing the entire body as well. The treatment subject can select a treatment course other than the blood circulation improvement treatment course. In addition, in this example, determination is made based on only the body information from the sphygmomanometer 31. However, in addition to the blood pressure value, the treatment course may be selected based on the body information of the pulse value. The screen 21 in this state has a [health support] button 28 displaying the health information notification portion 26 (will be described later). When the [health support] button 28 is pressed, a detailed diagnosis result (will be described later) given by a physician can be displayed.

In this manner, in accordance with the blood pressure value of the treatment subject measured by using the sphygmomanometer 31, the massage machine 10 can recommend the blood circulation improvement treatment course suitable for the treatment subject. In addition, since the blood pressure value exceeds the recommended usage range, the massage machine 10 can recommend that the massage machine 10 is not to be in use.

### Weight Management Treatment Course

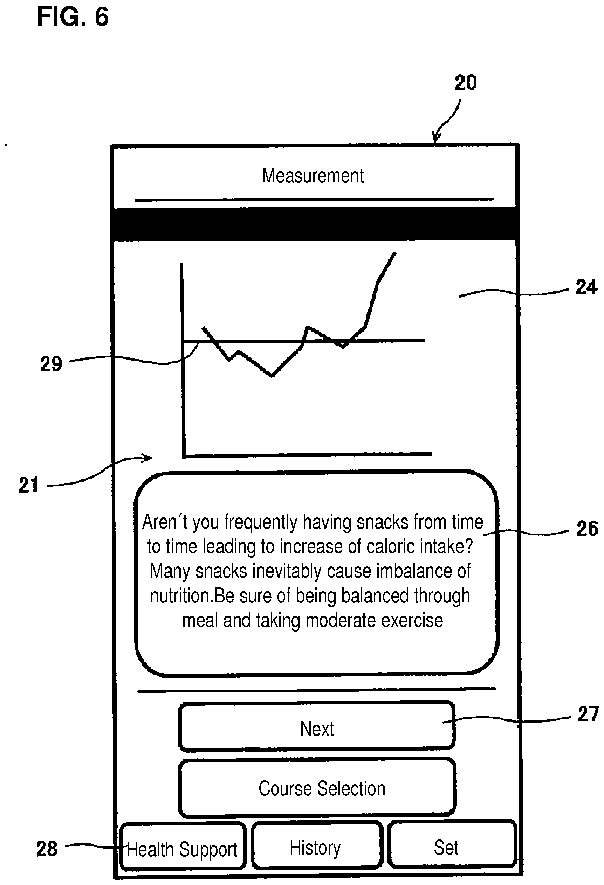

Fig. 6 is a front view illustrating an example of a screen for the measurement result displayed in the screen 21 of the operation unit 20. In this example, description will be given regarding an example in which the treatment subject sets a target weight 29 in advance and the target weight 29 is set as a standard. In this example, a result of a measurement performed by the treatment subject using the weight scale 32 before treatment is received by the control unit 17 of the massage machine 10, and the result is graphically displayed in an upper portion of the screen 21 of the operation unit 20 including chronological information of a weight (body information) of the treatment subject recorded in the storage portion 16. In this example, the weight is displayed so as to be grasped whether the weight is gained or lost with respect to the target weight 29.

In this manner, in a case where the treatment subject uses the weight scale 32 as the measurement portion 30 and the body information of the treatment subject is the weight, a change in the weight is shown. Accordingly, the treatment subject can grasp the change in his/her own body information through the operation unit 20. The portion of displaying information related to the weight is the weight information notification portion 24. In this example, a part the screen 21 serves as the weight information notification portion 24. This example illustrates a case where the weight measured by the weight scale 32 is significantly gained.

In addition, below the weight information notification portion 24, advice given based on a change in the weight is displayed. For example, the advice can be notified while including a diagnosis result from a physician (will be described later). In this manner, the portion of notifying the treatment subject of advice and the like given based on the body information of the treatment subject is the health information notification portion 26. In this example, a part of the screen 21 serves as the health information notification portion 26. Accordingly, the treatment subject can check suitable advice on the health management based on the change in the weight. When using the massage machine 10, the treatment subject can grasp activity to be performed for his/her own health management from the information of the health information notification portion 26.

When the [Next] button 27 is pressed, the weight management treatment course is recommended as the suitable treatment course. The weight management treatment course can include a slim course for a case where the weight is gained and a healthy weight management course for a case where the weight is lost, in accordance with the weight of the treatment subject measured by using the weight scale 32. The treatment subject can select a treatment course other than the weight management treatment course. The screen 21 in this state has the [health support] button 28 displaying the health information notification portion 26 (will be described later). When the [health support] button 28 is pressed, a detailed diagnosis result (will be described later) given by a physician can be displayed.

The slim course can include treatment of stimulating an acupuncture point for suppressing appetite. In the slim course, an appropriate weight loss curve can be shown to a person who desires to lose weight. For example, the treatment subject sets a target weight and a target time limit. In contrast, the massage machine 10 displays advice such that suitable weight-loss can be achieved with respect to the target weight. As the advice, the weight loss curve to the target time limit can be shown trough graphic display.

The healthy weight management course can include treatment of stimulating an acupuncture point for promoting appetite. In the healthy weight management course, the height and the weight of the treatment subject are measured by using the measurement portion 30. Then, the BMI value of the treatment subject with respect to a standard BMI value (physique index) is displayed, and advice thereof is displayed. In a case where the BMI value is higher than the standard, treatment of stimulating an acupuncture point for being slim can be included. The change in the BMI can be recorded and can be graphically displayed. During the management, when the BMI value deviates from an appropriate range, advice can be displayed in the operation unit 20. In addition to the measurement result of the weight scale 32, the body fat percentage, the moisture quantity, and the muscle mass can be managed by using a body composition meter.

In this manner, according to the treatment system 1, when the treatment subject measures the body information by using the measurement portion 30, the massage machine 10 receives the body information and recommends a treatment course suitable for the body information of the treatment subject to the treatment subject. Thus, the treatment subject can easily recognize the treatment course suitable for his/her own body information and can decide the treatment course.

### Configuration Related to Alarm

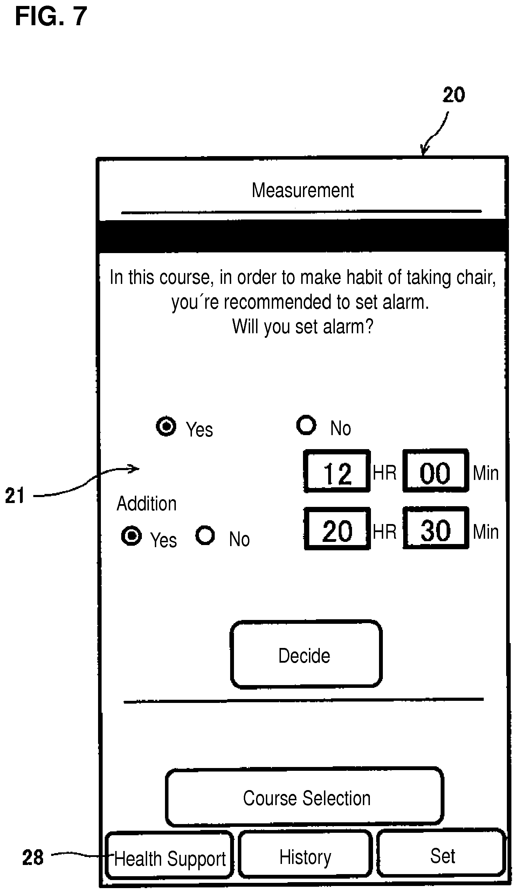

Fig. 7 is a front view illustrating an example of the screen for setting an alarm of the operation unit 20. As illustrated in Fig. 3, the massage machine 10 includes the alarm setting portion 18 and the timepiece portion 19. As illustrated in Fig. 7, in the screen 21 of the operation unit 20, the time for alarm can be set. The illustrated screen for setting an alarm can be displayed by using a [set] button of the screen 21. This example shows a screen in which an alarm is set so as to be emitted twice a day, in the morning and evening. When the alarm is set in the massage machine 10, it is possible to inform the treatment subject such that the body information is measured by the measurement portion 30 at a regular time.

As an alarm, for example, in a case of the treatment subject who often measures the body information by using the sphygmomanometer 31, the alarm notification portion 25 can inform the treatment subject of the using time by emitting an alarm twice a day (morning and evening). In addition, for example, in a case of the treatment subject who often measures the body information by using the weight scale 32, the alarm notification portion 25 can inform the treatment subject of the using time by emitting an alarm once a day at a regular time. The treatment subject can conduct health maintenance by using the measurement portion 30 measuring the body information at a regular time, through an alarm emitted from the alarm notification portion 25.

As an alarm emitted by the alarm notification portion 25, the treatment subject can be informed of an alarm through at least any one of light, a sound, vibration, and a screen display. As the alarm notification portion 25, the screen 21 of the operation unit 20 may have the function thereof. In a case where the screen 21 of the operation unit 20 has the function of the alarm notification portion 25, when it becomes the time set for an alarm, the screen 21 displays the time and the time can be informed through a sound. In addition, the time for alarm can also be informed by the operation unit 20 through a voice announcement. As the alarm notification portion 25, an LED light may be provided in the operation unit 20 or the massage machine 10 and the LED light may be turned on at the time set for an alarm. The method may be adopted alone or adopted by combining a plurality thereof.

In a case where the alarm notification portion 25 is the LED light, the color of the LED light may be caused to be different depending on a plurality of users. For example, in a case where a family uses the massage machine 10, it is possible to know whose alarm that is depending on the color. In addition, in a case where the alarm notification portion 25 is a voice speaker, a notification sound, a voice announcement, or the like can be registered in advance. In addition, in a case where the alarm notification portion 25 is displayed in the screen 21 of the operation unit 20, the screen 21 can display whose alarm that is and the treatment subject can be notified of the time for alarm.

When such an alarm setting portion 18 is provided, the treatment subject can easily know the time for alarm of the massage machine 10 through any of light, a sound, vibration, and a screen display. Moreover, in a case where a plurality of treatment subjects use the massage machine 10, each of the plurality of treatment subjects can easily know his/her own time for alarm by causing the color of the light, the sound, the vibration, and the screen display to be different depending on the treatment subject.

### Another Embodiment (Advice on Health Information)

As illustrated in Fig. 1, the massage machine 10 and the service provider 40 are connected to each other via the communication network 3. Therefore, the service provider 40 can obtain the body information of the treatment subject acquired by the massage machine 10 from the peripheral measurement portion 30 (sphygmomanometer 31, pulse wave detector, weight scale 32, body fat scale, or the like) via the communication network 3.

Meanwhile, in the treatment system 1 of the embodiment, the service provider 40 and the hospital 50 are connected to each other via the communication network 3. Therefore, the service provider 40 provides the hospital 50 with the body information of the treatment subject obtained from the massage machine 10, and diagnosis such as evaluation and advice with respect to the body information of the treatment subject can be received from the hospital 50.

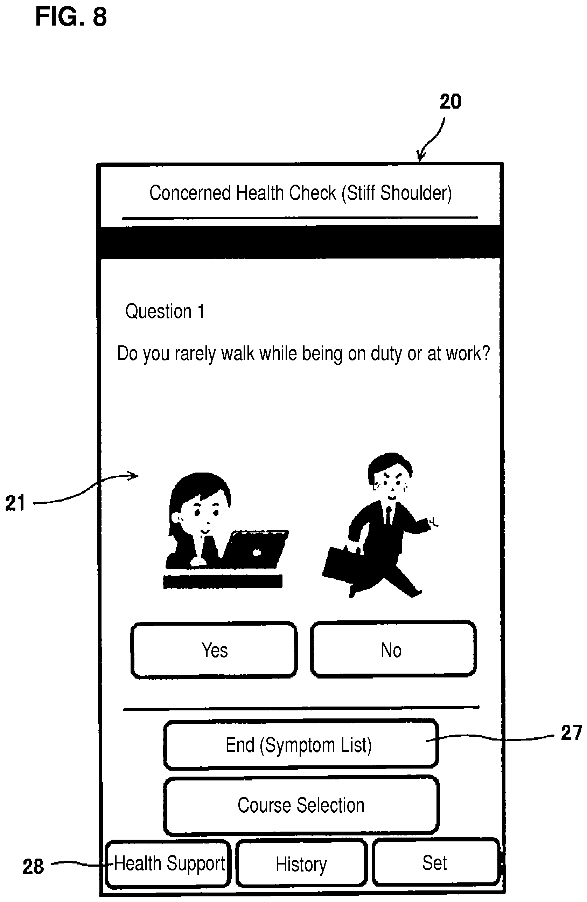

In addition, as illustrated in Fig. 8, the control unit 17 of the massage machine 10 can ask the treatment subject a question related to health information through the screen 21 of the operation unit 20, and the treatment subject can answer the question. The illustrated example is an example of Question 1 related to "stiff shoulder". The process can proceed to the next Question 2 and so on by pressing "YES" or "NO". As the questions, questions such as "lumbago", "stress", and the like can be included. When the "END" button 27 is pressed, a symptom list can be displayed. The result of the question is transmitted to the communication network 3 by the control unit 17, and the hospital 50 can receive the result of the question from the communication network 3. The hospital 50 can conduct the evaluation and advice with respect to the result of the question.

The diagnosis result (evaluation and advice) prepared by the hospital 50 is sent to the service provider 40 via the communication network 3. The service provider 40 transmits the diagnosis result to the massage machine 10 of the treatment subject. The diagnosis result can include the advice related to health information of the treatment subject acquired from the service provider 40. In this example, the service provider 40 performs a relay between the massage machine 10 and the hospital 50 such that the body information and the diagnosis result (evaluation and advice) are transmitted and received therebetween. However, the body information and the diagnosis result may be directly transmitted and received between the massage machine 10 and the hospital 50. In this case, the diagnosis result (evaluation and advice) can be transmitted from the hospital 50 to the communication network 3, and the control unit 17 of the massage machine 10 can receive the evaluation and advice from the communication network 3. The information may be temporarily stored in the communication server 41.

The massage machine 10 issues a notification of the received diagnosis result (evaluation and advice) or the like through the health information notification portion 26. When the treatment subject presses the [health support] button 28 in Figs. 5 and 6, a detailed diagnosis result can be displayed in the screen 21. Accordingly, the treatment subject can check the diagnosis result or the advice of the physician with respect to his/her own body information in the massage machine 10. For example, in a case where the hospital 50 is at a distant place, the treatment subject can check the diagnosis result or the advice of the physician at his/her home. In addition, the treatment subject can receive the evaluation and advice from the hospital 50 with respect to the result of the question related to the health information conducted in the massage machine 10, and the evaluation and advice can be checked in the massage machine 10.

Moreover, the massage machine 10 may include a camera (not illustrated) imaging the treatment subject, and a microphone (not illustrated) converting a voice of the treatment subject into an electric signal. For example, the camera and the microphone may be included in the operation unit 20. The screen 21 of the operation unit 20 serving as the health information notification portion 26 can also serve as a display portion imaging the physician of the hospital 50 via the communication network 3. In this manner, the treatment subject and the physician can have an interview via the screen 21 of the health information notification portion 26. Accordingly, the treatment subject can have an interview with the physician at a separated place via the health information notification portion 26 of the massage machine 10. The treatment subject can directly select the treatment course and decide the strength of the massage force based on the diagnosis result or the advice.

Transmitting and receiving of the data between the service provider 40 and the hospital 50 are not necessarily performed via the communication network 3. For example, the body information of the treatment subject is stored in a storage medium (for example, a USB memory) and is transmitted from the service provider 40 to the hospital 50. In the hospital 50, diagnosis is performed based on the body information of the treatment subject, and the diagnosis result thereof is stored in the storage medium. The storage medium is caused to return to the service provider 40. The service provider 40 may transmit the diagnosis result stored in the storage medium to the massage machine 10 of the treatment subject. The service provider 40 and the hospital 50 may be one body.

### Further another Embodiment (Usage Check for Massage Machine)

As illustrated in Fig. 1, the massage machine 10 is connected to the communication network 3. Therefore, the control unit 17 of the massage machine 10 can transmit the body information of the treatment subject to a registered transmission destination which is registered in advance, via a communication network, in addition to the service provider 40 and the communication server 41. The registered transmission destination can be a registerer terminal and the like to which the body information of the treatment subject is desired to be transmitted in addition to the service provider 40 managing the massage machine 10, and the communication server 41.

In this manner, when the treatment subject measures the body information by using the measurement portion 30 and the control unit 17 of the massage machine 10 receives the body information thereof, the body information of the treatment subject can be transmitted to the registered transmission destination via the communication network 3. The timing of transmitting the body information of the treatment subject can be the time the control unit 17 receives the body information of the treatment subject, a time set in advance, or the like. For example, by adopting the time set in advance, the result of the body information measured by the treatment subject can be transmitted to the registerer terminal at the set time.

Accordingly, when a portable terminal or the like (portable telephone or the like) of a child is set as the registered transmission destination of the massage machine 10 used by a parent, the child living at a separated place can receive and check the body information of the parent who is the treatment subject. Moreover, in this manner, "watching" of checking the parent who uses the massage machine 10 can be performed by the child at a distant place. When the registered transmission destination is the communication server 41, the child can check a change or the like in the body information of the parent from a distant place by storing the body information of the treatment subject in the communication server 41, for example. When the registered transmission destination is the service provider 40, the treatment subject using the massage machine 10 can be checked on the side of the service provider 40. In this manner, "watching" of checking the treatment subject using the massage machine can be performed by the service provider.

### General

As described above, according to the treatment system 1 including the massage machine 10, when the body information is measured by using the measurement portion 30 such as the sphygmomanometer 31 and the weight scale 32 before treatment, the massage machine 10 can receive the body information and can recommend a treatment course suitable for the body information of the treatment subject. Accordingly, the treatment subject can easily recognize the treatment course suitable for his/her own body information and can decide the treatment course.

In addition, based on the body information of the treatment subject, the control unit 17 weakens the massage force or starts treatment in a state where a part of the treatment operation is turned off. Accordingly, controlling can be performed such that the treatment operation suitable for the body information of the treatment subject is performed.

Moreover, when an alarm is emitted at the time set through the alarm setting portion 18 of the massage machine 10, the treatment subject can perform a measurement using the peripheral instruments at a regular time and can continue the health management.

In the embodiments, description has been given regarding an example of the massage machine 10 which has the seat portion 11 and the backrest portion 12 and in which the backrest portion 12 can be erected and tilted. However, the present invention can also be applied to a massage machine in which the backrest portion 12 has the fixed angle, and a massage machine other than the chair-type massage machine. The type of the massage machine 10 is not limited to the embodiments described above.

In addition, in the embodiments, description has been given regarding an example in which the massage machine 10 is connected to the communication server 41, the service provider 40, and the hospital 50 via the communication network 3. However, the treatment system 1 is acceptable as long as the treatment system 1 has a configuration in which the body information of the peripheral instruments (sphygmomanometer 31, weight scale 32, and the like) related to the treatment subject can be received by the massage machine 10. The treatment system 1 is not limited to the configuration including all of the configurations of the embodiments.

Moreover, the embodiments described above are examples, and the communication server 41 may be integrated with the service provider 40 or may be a server managed by a different service provider. Various changes can be made within the scope not harming the gist of the present invention, and the present invention is not limited to the embodiments described above.

### LIST OF REFERENCE NUMERALS

- 1: TREATMENT SYSTEM
- 2: RADIO COMMUNICATION
- 3: COMMUNICATION NETWORK
- 10: MASSAGE MACHINE
- 11: SEAT PORTION
- 12: BACKREST PORTION
- 15: TREATMENT PORTION (ROLLER UNIT)
- 16: STORAGE PORTION
- 17: CONTROL UNIT
- 18: ALARM SETTING PORTION
- 19: TIMEPIECE PORTION
- 20: OPERATION UNIT
- 21: SCREEN
- 22: TREATMENT COURSE NOTIFICATION PORTION
- 23: BLOOD PRESSURE INFORMATION NOTIFICATION PORTION
- 24: WEIGHT INFORMATION DISPLAY PORTION
- 25: ALARM NOTIFICATION PORTION
- 26: HEALTH INFORMATION NOTIFICATION PORTION
- 27: [END] BUTTON
- 28: [HEALTH SUPPORT] BUTTON
- 30: MEASUREMENT PORTION
- 31: SPHYGMOMANOMETER
- 32: WEIGHT SCALE
- 40: SERVICE PROVIDER
- 41: COMMUNICATION SERVER
- 50: HOSPITAL (MEDICAL FACILITY)
- 60: TERMINAL

## Claims

1. A treatment system including a massage machine, comprising:
a measurement portion which measures body information of a treatment subject before treatment;
a massage machine which has a treatment portion treating the treatment subject, a control unit controlling the treatment portion, and a storage portion storing a plurality of treatment courses having treatment contents for the treatment portion different from each other; and
a treatment course notification portion which recommends the treatment courses to the treatment subject,
wherein the measurement portion and the control unit are connected to each other through a cable or by radio,
wherein the control unit is configured to receive the body information of the treatment subject from the measurement portion through a cable or by radio, and to select a suitable treatment course from the plurality of treatment courses stored in the storage portion, based on the body information such that the treatment course notification portion recommends the selected treatment course to the treatment subject.

2. The treatment system including a massage machine according to Claim 1,
wherein the control unit is configured to be able to change at least any one of a massaging force and a part of a treatment operation in the treatment course based on the body information of the treatment subject.

3. The treatment system including a massage machine according to Claim 1 or 2,
wherein the measurement portion has at least a sphygmomanometer,
wherein the plurality of treatment courses include at least a blood circulation improvement treatment course for improving blood circulation, and
wherein the control unit is configured to determine whether or not to select the blood circulation improvement treatment course as the suitable treatment course, based on the body information of the treatment subject from the sphygmomanometer.

4. The treatment system including a massage machine according to Claim 3, further comprising:
a blood pressure information notification portion,
wherein the control unit is configured to cause the body information of the treatment subject from the sphygmomanometer to be chronologically recorded in the storage portion and to cause the blood pressure information notification portion to issue a notification of the chronological body information recorded in the storage portion.

5. The treatment system including a massage machine according to Claim 1 or 2,
wherein the measurement portion has at least a weight scale,
wherein the plurality of treatment courses include at least a weight management treatment course for managing a weight, and
wherein the control unit is configured to determine whether or not to select the weight management treatment course as the suitable treatment course, based on the body information of the treatment subject from the weight scale.

6. The treatment system including a massage machine according to Claim 5, further comprising:
a weight information notification portion,
wherein the control unit is configured to cause the body information of the treatment subject from the weight scale to be chronologically recorded in the storage portion and to cause the weight information notification portion to issue a notification of the chronological body information recorded in the storage portion.

7. The treatment system including a massage machine according to any one of Claim 1 to 6, further comprising:
an information terminal which is provided between the measurement portion and the control unit and in which the body information of the treatment subject is recorded,
wherein the information terminal is configured to be able to communicate with the control unit through a cable or by radio.

8. The treatment system including a massage machine according to any one of Claim 1 to 7,
wherein the massage machine has at least a seat portion in which the treatment subject takes a seat, and a backrest portion which supports the upper half of the body of the treatment subject who takes a seat in the seat portion, from the back, and
wherein the control unit is configured to set an erect/tilt angle of the backrest portion in accordance with the treatment course.

9. The treatment system including a massage machine according to any one of Claim 1 to 8,
wherein the massage machine has a timepiece portion, and
wherein the control unit is configured to cause an alarm notification portion to issue a notification of an alarm at a predetermined time based on time information from the timepiece portion.

10. The treatment system including a massage machine according to Claim 9,
wherein the alarm notification portion is configured to issue a notification of an alarm through at least one of light, a sound, vibration, and a screen display.

11. The treatment system including a massage machine according to any one of Claim 1 to 10,
wherein the control unit is configured to recommend that the massage machine is not to be in use in a case where the body information of the treatment subject exceeds a recommended usage range.

12. The treatment system including a massage machine according to any one of Claim 1 to 11,
wherein the control unit is configured to cause a health information notification portion to issue a notification of advice related to health information prepared based on the body information of the treatment subject.

13. The treatment system including a massage machine according to Claim 12,
wherein the massage machine is connected to a hospital via a communication network,
wherein the control unit asks the treatment subject a question related to the health information and transmits a result of the question to the communication network,
wherein the hospital receives the result of the question from the communication network and transmits evaluation and advice with respect to the result of the question to the communication network, and
wherein the control unit is configured to receive the evaluation and advice from the communication network and to cause the health information notification portion to issue a notification.

14. The treatment system including a massage machine according to Claim 13,
wherein the massage machine has a camera imaging the treatment subject, and a microphone converting a voice of the treatment subject into an electric signal,
wherein the health information notification portion also serves as a display portion imaging a physician of the hospital via the communication network, and
wherein the health information notification portion is configured to allow the treatment subject and the physician to have an interview.

15. The treatment system including a massage machine according to any one of Claim 1 to 10,
wherein the massage machine is connected to a registered transmission destination via the communication network, and
wherein the control unit is configured to transmit the body information of the treatment subject to the registered transmission destination via the communication network.
